(19) Europäisches Patentamt
European Patent Office
Office européen des brevets

(11) Veröffentlichungsnummer : **0 060 456**
**B1**

(12) # EUROPÄISCHE PATENTSCHRIFT

(45) Veröffentlichungstag der Patentschrift : **19.12.84**

(21) Anmeldenummer : **82101715.9**

(22) Anmeldetag : **05.03.82**

(51) Int. Cl.³ : **C 07 D233/06**, C 07 D239/06, C 23 F 11/14

(54) **Inhibitoren gegen die Korrosion von H2S und CO2 in Wasser-in-Öl-Emulsionen.**

(30) Priorität : **14.03.81 DE 3109826**

(43) Veröffentlichungstag der Anmeldung : **22.09.82 Patentblatt 82/38**

(45) Bekanntmachung des Hinweises auf die Patenterteilung : **19.12.84 Patentblatt 84/51**

(84) Benannte Vertragsstaaten : **DE FR GB NL**

(56) Entgegenhaltungen :
**DE-A- 2 133 723**
**US-A- 4 101 441**
**US-A- 4 134 959**
**US-A- 4 238 350**

(73) Patentinhaber : **BASF Aktiengesellschaft**
**Carl-Bosch-Strasse 38**
**D-6700 Ludwigshafen (DE)**

(72) Erfinder : **Oppenlaender, Knut, Dr. Chem.**
**Otto-Dill-Strasse 23**
**D-6700 Ludwigshafen (DE)**
Erfinder : **Stork, Karl, Dr. Chem.**
**Reutersgarten 1**
**D-6840 Lampertheim (DE)**
Erfinder : **Barthold, Klaus, Dr. Chem.**
**Paulusbergstrasse 4**
**D-6800 Mannheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 060 456 B1

**Beschreibung**

Die Erfindung betrifft neuartige Inhibitoren bzw. Inhibitorsysteme gegen die Korrosion von $H_2S$ und $CO_2$ in Wasser-in-Öl-, insbesondere Salzwasser-in-Öl-Emulsionen.

Es ist bekannt und geübte Praxis, bei der Ölförderung, dem Transport und der Lagerung des Rohöls durch Injizieren eine Lösung oder Dispersion von Korrosionsinhibitoren in das Öl einzubringen, so daß sich auf der Oberfläche der mit dem Öl in Berührung kommenden Metallteile eine Schutzschicht ausbildet. Die Erdölemulsionen enthalten zumeist Salzwasser und in vielen Fällen — bedingt durch die Provenienz des Öls — $H_2S$ und $CO_2$, welche stark korrodierend wirken.

Die Inhibitoren hierfür sollen in Öl löslich und in Salzwasser zumindest dispergierbar sein, damit sie optimal wirken können.

Aus der DE-OS 28 46 977 sind derartige Systeme bekannt. Es handelt sich um Imidazolinium-Salze, die gelöst in einem öllöslichen organischen Lösungsmittel und in Gegenwart eines Kohlenwasserstofföls zur Anwendung gelangen.

Der Nachteil besteht in der relativ komplizierten Dosierungsform (3 Komponenten) und vor allem darin, daß der Korrosionsschutz noch nicht in allen Fällen befriedigt. Wir haben beispielsweise festgestellt, daß das in der genannten Publikation beschriebene Imidazolin der Formel

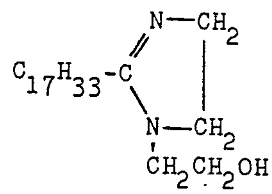

(als Chlorid) für sich allein keine genügende Wirkung gegen korrosiven Abtrag durch die Einwirkung von $H_2S$ entfaltet.

Das Ziel der Erfindung bestand nun darin, solche organische Verbindungen aufzufinden, die optimal gegen die $H_2S$- und $CO_2$-Korrosion in (Salz)wasser-in-Öl-Emulsionen wirken, und die in beiden Phasen ohne zusätzliche Lösungsmittelhilfe gleichmäßig verteilbar sind.

Dieses Ziel wurde mit Verbindungen erreicht, wie sie gemäß dem Anspruch 1 definiert sind. Es handelt sich um Amide cyclischer Formamidine, die ein Imidazolin- oder 3,5-Tetrahydro-pyrimidinsystem darstellen können.

Die Verbindungen sind in einfacher Weise durch klassische Reaktionen erhältlich.

Man setzt zunächst eine Carbonsäure R—COOH, wobei R einen $C_7$- bis $C_{25}$-, vorzugsweise $C_8$- bis $C_{17}$-Alkyl- oder Alkenylrest bedeutet, mit Diethylentriamin oder Di-n-propylentriamin bei 120 bis 160 °C innerhalb von 5 bis 10 Stunden im Molverhältnis 1 : 1 bis 1 : 1,2 um und setzt das erhaltene Säureamid bei 120 bis 150 °C innerhalb 0,5 bis 2 Stunden mit Formamid um. Anschließend wird bei 200 bis 300 °C innerhalb 5 bis 10 Stunden vorzugsweise unter Vakuum (1,5 bis 80 mbar) kondensiert.

Die erhaltenen Imidazolin- bzw. Tetrahydro-pyrimidinderivate können in reiner Form isoliert werden.

Als Ausgangscarbonsäuren kommen alle in Betracht, wie sie oben definiert sind. Vorzugsweise wählt man z. B. n-Octansäure, iso-Octansäure (2-Ethylhexansäure), i-Nonansäure (3,5,5-Trimethylhexansäure), Laurinsäure, Stearinsäure, Ölsäure, Behensäure und Gemische davon. Auch Gemische natürlicher Fettsäuren, wie Talgfett-, Kokosfett-, Rübölfett- und Palmkernfettsäure sind geeignet.

Die Verbindungen wirken für sich allein als Inhibitoren gegen die $H_2S$- und $CO_2$-Korrosion schon sehr befriedigend, d. h. der Abtrag nach dem sog. « Wheel »-Test (dynamischer Test) geht gegenüber dem Blindwert auf unter ein Viertel dieses Wertes zurück.

Eine Steigerung kann noch erreicht werden, wenn bezogen auf die Imidazolin- bzw. Tetrahydro-pyrimidinderivate 0,5 bis 100 Gew.-%, vorzugsweise 1 bis 30 Gew.-% an kolloidalem Schwefel anwesend sind. Die Einarbeitung des Schwefels in das System kann durch bloßes Zumischen « kolloidalen » Schwefels, oder auch (vorzugsweise) durch Erhitzen des Gemisches auf 100 bis 200 °C innerhalb von 1 bis 3 Stunden erfolgen.

Derart erhaltene Systeme vermögen den Abtrag auf weniger als ein Fünftel des Blindwertes herabzudrücken.

Die Inhibitoren(gemische) werden den Wasser-in-Öl-Emulsionen in Mengen von 50 bis 1 000 ppm — bezogen auf das Gewicht der Emulsion — zugesetzt. Sie verteilen sich ohne Schwierigkeiten sowohl in der wäßrigen als auch in der Ölphase.

Die folgenden Beispiele erläutern die Erfindung.

## Beispiele

A) Herstellung :

a) 423,7 g Ölsäure werden auf 70 bis 80 °C erwärmt und bei dieser Temperatur 170,3 g

Diethylentriamin zugetropft ; dann wird 7 bis 8 Stunden bei 150 bis 160 °C Reaktionswasser abdestilliert. Zum gebildeten Säureamid wird bei 120 bis 150 °C innerhalb 1 Stunde 67,6 g Formamid zugetropft. Anschließend wird bei 200 bis 250 °C und ca. 20 Torr Vakuum 8 Stunden kondensiert (Verbindung I, R = $C_{17}H_{33}$).

Ausbeute : 565 g braunes viskoses Öl.

b) 316,4 g Isononansäure (3,5,5-Trimethylhexansäure) werden auf 70 bis 80 °C erwärmt und bei dieser Temperatur 288,7 g Dipropylentriamin zugetropft ; dann wird 7 bis 8 Stunden bei 150 bis 160 °C Reaktionswasser abdestilliert. Zum gebildeten Säureamid wird bei 120 bis 150 °C innerhalb 30 Minuten 90 g Formamid zugetropft. Anschließend wird bei 200 bis 250 °C und ca. 20 Torr Vakuum unter Ammoniak-Abspaltung kondensiert.

Ausbeute : 560 g braunes viskoses Öl. (Verbindung II, R = $C_8H_{17}$).

B) Anwendung :

Die Korrosionsinhibitoren wurden nach dem sog. dynamischen oder « Wheel »-Test geprüft. Es ist eine bei der Erdölförderung gängige Methode zur Prüfung von Inhibitoren.

Die Testcoupons sind Eisenbleche der Abmessung (130 mm × 10 mm × 1 mm). Diese werden geschmirgelt, mit Toluol entfettet und gewogen. Als Testmedium verwendeten wir Testbenzin, das 50 Gew.-% Salzwasser mit 3 % NaCl-Gehalt einemulgiert enthielt. Das Testmedium wurde zur Simulation der Feldbedingungen mit $H_2S + CO_2$ gesättigt und in Testflaschen abgefüllt. Dann wurden die zu prüfenden Inhibitoren in Mengen von 250 ppm eingegeben. Die Testblechstreifen wurden an den Flaschendeckeln befestigt und in die Testmedien eingehängt.

Danach wurden die Testflaschen auf einer rotierenden Achse (Wheel) befestigt, die sich mit 40 UpM in einem auf 80 °C gehaltenen Wasserbad drehte. Die Dauer der Versuche betrug 16 Stunden.

Danach wurden die Teststreifen mit einer inhibierenden Säure gereinigt, entfettet, getrocknet und zur Bestimmung des Gewichtsverlustes gewogen. Die Auswertung erfolgt im Vergleich zum Blindwert (Versuch ohne Inhibitorzusatz).

Die Ergebnisse sind aus der folgenden Tabelle ersichtlich.

Tabelle

Verbindung I

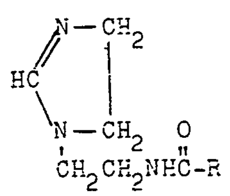

Verbindung II

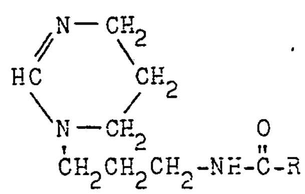

Dosierung: 250 ppm

| Verbindung | R | Abtrag [mg/coupon] |
|---|---|---|
| I | $C_{17}H_{33}$ | 22,9 |
| I | $C_{17}H_{35}$ | 22,5 |
| II | $C_{17}H_{33}$ | 22,4 |
| II | $C_{17}H_{35}$ | 21,1 |
| I + 1/5/10 % S | $C_{17}H_{33}$ | 21,9/21,2/17,4 |
| I + 1/5/10 % S | $C_{17}H_{35}$ | 22,4/22,7/26,9 |
| II + 1/5/10 % S | $C_{17}H_{33}$ | 28,6/26,9/23,3 |
| I | i-$C_7H_{17}$ | 35,5 |
| I | i-$C_8H_{17}$ | 33,8 |
| I | $C_{11}H_{23}$ | 28,2 |
| I | $C_{17}H_{33}$ | 22,9 |
| I | $C_{17}H_{35}$ | 22,5 |

Tabelle (Fortsetzung)

| Verbindung | R | Abtrag [mg/coupon] |
|---|---|---|
| I | Talgfettsäurerest | 26,4 |
| I | Kokosfettsäurerest | 24,9 |
| I | Rohölfettsäurerest | 20,2 |
| I | Palmkernfettsäurerest | 23,4 |
| II | i-$C_7H_{15}$ | 34,8 |
| II | i-$C_8H_{17}$ | 29,3 |
| II | $C_{11}H_{33}$ | 27,1 |
| II | $C_{17}H_{33}$ | 21,1 |
| II | $C_{17}H_{33}$ | 22,4 |
| II | Talgfettsäurerest | 24,2 |
| II | Kokosfettsäurerest | 26,1 |
| II | Rübölfettsäurerest | 21,3 |
| II | Palmkernfettsäurerest | 22,7 |
| Blindwert (ohne Schwefel) | | 98,3 |
| " (mit 10 % S) | | 114,9 |
| Vergleich | | |
| 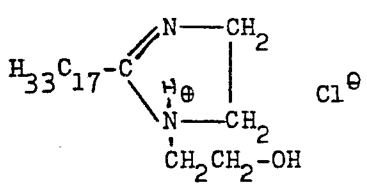 | | 52,8 |

gemäß DE-OS 28 46 977.

**Ansprüche**

1. Verbindungen der Formel I

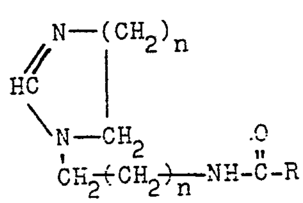

(I)

in der R einen $C_7$- bis $C_{25}$-Alkyl- oder Alkenylrest und n die Zahlen 1 oder 2 bedeuten.

2. Verwendung von Verbindungen gemäß Anspruch 1 als Inhibitoren gegen die Korrosion von $H_2S$ und $CO_2$ in Wasser-in-Öl-Emulsionen.

3. Verwendung von Verbindungen der Formel I gemäß Anspruch 2, dadurch gekennzeichnet, daß zusätzlich — bezogen auf die Verbindungen der Formel I — 0,5 bis 100 Gew.-% an elementarem Schwefel miteingesetzt werden.

**Claims**

1. A compound of the formula I

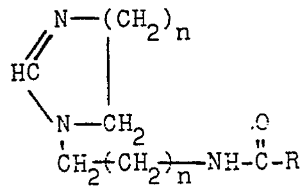

(I)

where R is alkyl or alkenyl, each having 7 to 25 carbon atoms, and n is 1 or 2.

2. The use of a compound as claimed in claim 1 as an inhibitor for the prevention of corrosion caused by $H_2S$ and $CO_2$ in water-in-oil emulsions.

3. The use of a compound of the formula I, as claimed in claim 2, wherein from 0.5 to 100 % by weight, based on the compound of the formula I, of elementary sulphur is additionally employed.

**Revendications**

1. Composé répondant à la formule I

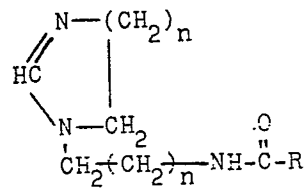

dans laquelle R représente un reste alcoylique ou alcénylique comportant 7 à 25 atomes de carbone et n le nombre 1 ou 2.

2. Application des composés selon la revendication 1 comme inhibiteurs contre la corrosion par $H_2S$ et $CO_2$ dans des émulsions huileuses, du type eau dans l'huile.

3. Application des composés répondant à la formule I selon la revendication 2, caractérisée en ce qu'on utilise en outre 0,5 à 100 % en poids de soufre élémentaire (le pourcentage se rapportant aux composés répondant à la formule I).